# EUROPEAN PATENT SPECIFICATION

(11) **EP 1 996 643 B1**
(45) Date of publication and mention of the grant of the patent: **24.02.2010**
(21) Application number: 07711258.9
(22) Date of filing: 07.03.2007
(51) Int. Cl.: C08G 63/664, C08G 81/00, C08J 9/00, C08J 9/28, A61L 27/18, A61L 15/26, A61L 27/56

(54) **DEGRADABLE HYDROPHILIC BLOCK COPOLYMERS WITH IMPROVED BIOCOM PATIBIL FOR SOFT TISSUE REGENERATION**
ABBAUBARE COPOLYMERE MIT EINEM HYDROPHILEN BLOCK MIT VERBESSERTER BIOKOMPATIBILITÄT FÜR DIE WEICHGEWEBEREGENERATION
COPOLYMÈRES A BLOC HYDROPHILES DÉGRADABLES PRESENTANT UNE BIOCOMPATIBILITÉ AMÉLIORÉE POUR LA RÉGÉNÉRATION DES TISSUS MOUS

(30) Priority: 09.03.2006 DK 200600337; 01.09.2006 DK 200601131; 26.10.2006 WO PCT/EP2006/067837
(43) Date of publication of application: 03.12.2008
(73) Proprietor: Coloplast A/S, 3050 Humlebæk (DK)
(72) Inventor: VANGE, Jakob, DK-3000 Helsingoer (DK); SCHWACH-ABDELLAOUI, Khadija, DK-2880 Bagsvaerd (DK); EVERLAND, Hanne, DK-2880 Bagsværd (DK); NIELSEN, Peter, Sylvest, DK-3500 Værløse (DK); NIELSEN, Brian, DK-3330 Gørløse (DK); JESPERSEN, Lene, Karin, DK-3480 Fredensborg (DK); NIELSEN, Lene, Feldskov, DK-1054 Copenhagen K (DK)
(74) Representative: Inspicos A/S
(86) International application number: PCT/DK2007/000115
(87) International publication number: WO 2007/101443

(56) References cited:
- EP-A1- 1 234 587
- EP-A2- 1 621 216
- US-A- 4 716 203
- US-A- 5 102 983
- US-A- 5 621 050
- US-A1- 2002 018 797
- US-B1- 6 562 374
- BELETSI A ET AL: "Biodistribution properties of nanoparticles based on mixtures of PLGA with PLGA-PEG diblock copolymers" INTERNATIONAL JOURNAL OF PHARMACEUTICS, AMSTERDAM, NL, vol. 298, no. 1, 14 July 2005 (2005-07-14), pages 233-241, XP004943756 ISSN: 0378-5173
- WITT C ET AL: "The degradation, swelling and erosion properties of biodegradable implants prepared by extrusion or compression moulding of poly(lactide-co-glycolide) and ABA triblock copolymers" BIOMATERIALS, ELSEVIER SCIENCE PUBLISHERS BV., BARKING, GB, vol. 21, no. 9, May 2000 (2000-05), pages 931-938, XP004202459 ISSN: 0142-9612
- GREF R ET AL: "BIODEGRADABLE LONG-CIRCULATING POLYMERIC NANOSPHERES" SCIENCE, AMERICAN ASSOCIATION FOR THE ADVANCEMENT OF SCIENCE,, US, vol. 263, 18 March 1994 (1994-03-18), pages 1600-1603, XP002013571 ISSN: 0036-8075
- RONNEBERGER B., KAO W.J., ANDERSON J.M., KISSEL T.: "In vivo biocompatibility study of ABA triblock copolymers consisting of poly(lactic-co-glycolic acid) A blocks attache to central pol(oxyethylene) B Blocks" JOURNAL OF BIOMEDICAL MATERIALS RESEARCH, vol. 30, 1996, pages 31-40, XP002405091
- MORLOCK M.,KISSEL T., LI Y.X., KOLL H., WINTER G.: "Erythropoietin loaded microspheres prepared from biodegradable LPLG-PEO-LPLG triblock copolymers: protein stabilization and in-vitro release properties" JOURNAL OF CONTROLLED RELEASE, vol. 56, 1998, pages 105-115, XP002405092
- KIM K ET AL: "Incorporation and controlled release of a hydrophilic antibiotic using poly(lactide-co-glycolide)-based electrospun nanofibrous scaffolds" JOURNAL OF CONTROLLED RELEASE, ELSEVIER, AMSTERDAM, NL, vol. 98, no. 1, 23 July 2004 (2004-07-23), pages 47-56, XP004520545 ISSN: 0168-3659
- ZANGE R ET AL: "Biocompatibility testing of ABA triblock copolymers consisting of poly(L-lactic-co-glycolic acid) A blocks attached to a central poly(ethylene oxide) B block under in vitro conditions using different L929 mouse fibroblasts cell culture models" JOURNAL OF CONTROLLED RELEASE, ELSEVIER SCIENCE PUBLISHERS B.V., AMSTERDAM, NL, vol. 56, no. 1-3, 4 December 1998 (1998-12-04), pages 249-258, XP004153938 ISSN: 0168-3659
- SHIN K C ET AL: "A facile preparation of highly interconnected macroporous PLGA scaffolds by liquid-liquid phase separation II" POLYMER, ELSEVIER SCIENCE PUBLISHERS B.V, GB, vol. 46, no. 11, 11 May 2005 (2005-05-11), pages 3801-3808, XP004904279 ISSN: 0032-3861
- LEE S Y ET AL: "In vivo conjunctival reconstruction using modified PLGA grafts for decreased scar formation and contraction" BIOMATERIALS, ELSEVIER SCIENCE PUBLISHERS BV., BARKING, GB, vol. 24, no. 27, December 2003 (2003-12), pages 5049-5059, XP004464376 ISSN: 0142-9612

## Description

### FIELD OF THE INVENTION

The present invention relates to novel biodegradable polymers, to porous and other materials comprising such polymers, and to various medical uses of such materials.

### BACKGROUND OF THE INVENTION

Scaffolds are porous structures into which cells may be incorporated. They are usually made up of biocompatible, degradable materials and are added to tissue to guide the organization, growth and differentiation of cells in the process of forming functional tissue. The materials used can be either of natural or synthetic origin.

Poly(L-lactide) (PLLA), poly(D/L-lactide) (PDLLA) and poly(lactide-co-glycolide) (PLGA) have been known for a long time as degradable implant materials, and they are all FDA-approved for this purpose. They have been used as scaffolds for bone, cartilage, liver, skin, urethra, intestines, tendon and cardiovascular tissues.

A typical example of one of these applications is that the polymer is made into a porous structure, often by solvent casting/particle leaching. The structure is then pre-wetted with ethanol, and washed successively with water. This step is necessary because these polymers are hydrophobic, and an attempt to wet them directly with water fails. The wet structure is then seeded with cells and grown in a bioreactor before implantation.

Copolymers of polyethers and polyesters are also known. These are usually not used as scaffolds, as PEG-containing (polyethylene glycol) polymers are known to resist adhesion of cells and proteins. This class of polymers are used as carriers in drug delivery, where the high hydrophilicity and fouling-resistance of the polyether part is useful.

PLGA and copolymers of PEG and PLGA are known to have good biocompatibility in that they are non-toxic for cells and do not invoke inflammatory response in tissue. In Zange et al., Journal of Controlled Release, 56, 1998, 249-258, the biocompatibility of various PEG-PLGA copolymers are examined with in vitro models, and none show adverse effects of the polymers on mouse fibroblasts. For a polymer to perform in a scaffold, good biocompatibility is not enough. The cells have to efficiently adhere to the material. It is known that PEG-containing polymers and PEG-coated surfaces resist adhesion of cells and proteins.

US 6,201,072B1 teaches a group of PLGA-PEG-PLGA triblock copolymers with low molecular weight and distinct aqueous solubility characteristics for drug delivery applications.

WO 03000778 A1 uses (among other things) MPEG-PLGA (MPEG = methoxy-polyethylene glycol) with a linker in the OH-functional end for drug release purposes.

US 20040076673 discloses MPEG-PLGA with M_{w}<5000 for oral drug delivery.

CN 1446841 discloses three-dimensional porous frame materials of poly(lactide)-polyether block copolymers and a process for preparing such block copolymers.

The polymers for tissue engineering can be either natural or synthetic. The most widely used synthetic polymers are from the group of polyesters. The most common in this group are PLLA, PDLLA, PLGA, PCL (poly-ε-caprolactone), and various copolymers thereof. They are all hydrophobic materials, and initial adhesion of cells to scaffolds of these polyesters is sluggish at best.

The present inventors have found that by incorporating a hydrophilic block (i.e. a polyalkylene glycol block) in the polymer, the biocompatibility of polyesters is improved. This is due to better wetting characteristics of the material, and that initial cell adhesion is impaired on non-polar materials. Further, it has been found that by keeping the molar content of the polyalkylene units relative to the molar content of the lactide/glycolide units low, i.e. at the most 14 molar-%, superior polymers and derived materials are obtained.

### BRIEF DESCRIPTION OF THE INVENTION

One aspect of the present invention relates to a biodegradable polymer of the general formula:

A-O-(CH₂CH₂O)ₙ-methyl

wherein
A is a poly(lactide-co-glycolide) residue of a molecular weight of at least 8,000 g/mol, the molar ratio of (i) lactide units [-CH(CH₃)-COO-] and (ii) glycolide units [-CH₂-COO-] in the poly(lactide-co-glycolide) residue being in the range of 80:20 to 10:90, in particular 70:30 to 10:90,
n represents the average number of -(CH₂CH₂O)- units within a polymer chain and is an integer in the range of 16-250,
the molar ratio of (iii) polyethylene glycol units [-(CH₂CH₂O)-] to the combined amount of (i) lactide units [-CH(CH₃)-COO-] and (ii) glycolide units [-CH₂-COO-] in the poly(lactide-co-glycolide) residue(s) is in the range of 1:99 to 12:88,
and wherein the number average molecular weight of the copolymer is at least 20,000 g/mol.

Another aspect of the present invention relates to a biodegradable, porous material comprising a polymer of the general formula

A-O-(CHR¹CHR²O)ₙ-B

wherein
A is a poly(lactide-co-glycolide) residue of a molecular weight of at least 4000 g/mol, the molar ratio of (i) lactide units [-CH(CH₃)-COO-] and (ii) glycolide units [-CH₂-COO-] in the poly(lactide-co-glycolide) residue being in the range of 80:20 to 10:90,
B is either a poly(lactide-co-glycolide) residue as defined for A or is selected from the group consisting of hydrogen, C₁₋₆-alkyl and hydroxy protecting groups,
one of R¹ and R² within each -(CHR¹CHR²O)- unit is selected from hydrogen and methyl, and the other of R¹ and R² within the same -(CHR¹CHR²O)- unit is hydrogen,
n represents the average number of -(CHR¹CHR²O)- units within a polymer chain and is an integer in the range of 10-1000,
the molar ratio of (iii) polyalkylene glycol units [-(CHR¹CHR²O)-] to the combined amount of (i) lactide units [-CH(CH₃)-COO-] and (ii) glycolide units [-CH₂-COO-] in the poly(lactlde-co-glycolide) residue(s) is at the most 14:86,
and wherein the number average molecular weight of the copolymer is at least 10,000 g/mol.
wherein the porosity is at least 50%, such as in the range of 50-99.5%.

A third aspect of the present invention relates to a method for the preparation of a biodegradable, porous material of a polymer, the method comprising the steps of:
(a) dissolving a polymer as defined herein in a non-aqueous solvent so as to obtain a polymer solution;
(b) freezing the solution obtained in step (a) so as to obtain a frozen polymer solution; and
(c) freeze-drying the frozen polymer solution obtained in step (b) so as to obtain the biodegradable, porous material.

Further aspects of the present invention relates to a medical device element of a material comprising a polymer as defined herein; a medical device of a material comprising a polymer as defined herein; a biodegradable, porous material as defined herein for use in therapy, dentistry or surgery; the use of a biodegradable, porous material as defined herein for the preparation of a scaffold for supporting cell adhesion or the in-growth for regeneration of tissue; and the use of a biodegradable, porous material as defined herein for the preparation of a wound dressing.

### BRIEF DESCRIPTION OF THE FIGURES

Figure 1 illustrates MPEG-PLGA 2-30 kDa scaffolds supporting the development of reconstituted epidermis.

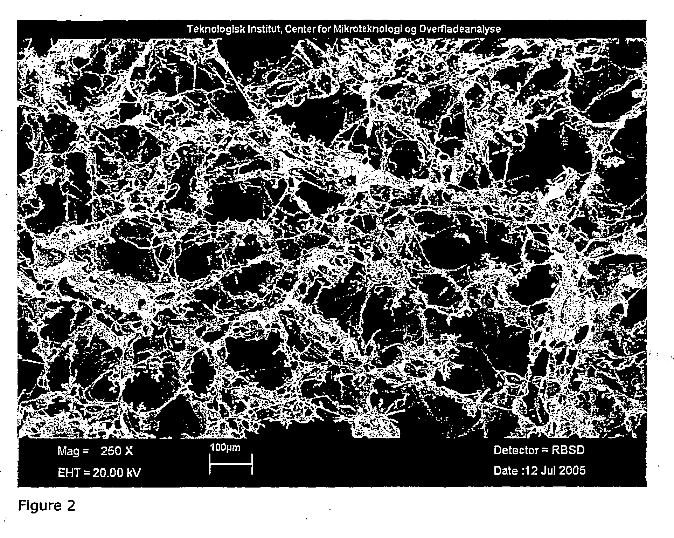

Figure 2: 1.5% (w/w) solution of a 2-30 50% lactide polymer (6% (w/w) MPEG) in dioxane, frozen at -5°C, then freeze-dried at -20°C.

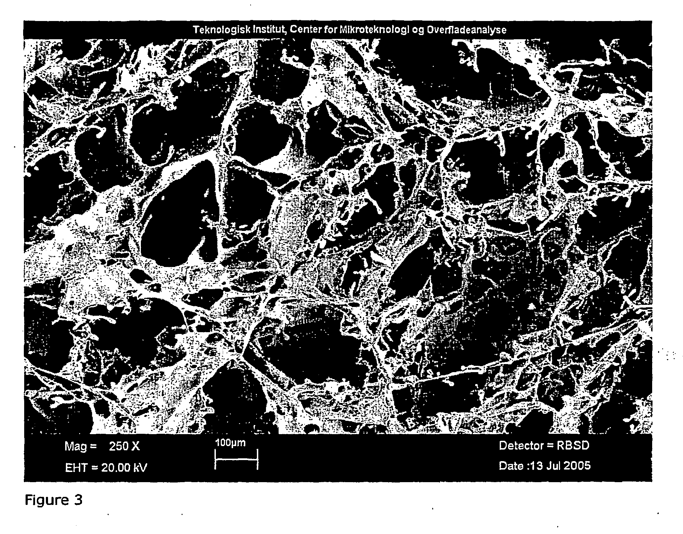

Figure 3: 1.5% (w/w) solution of a 2-30 50% lactide polymer (6% (w/w) MPEG) in dioxane, frozen at +5°C, then freeze-dried at -20°C.
Figure 4: SEM picture of freeze dried 1.5% MPEG-PLGA containing 40% ECM particles (magnification 250x).

### DETAILED DESCRIPTION OF THE INVENTION

The present inventors have found that particularly designed biodegradable polymers have highly interesting properties which can be utilised in scaffolds for supporting cell adhesion and/or the in-growth for regeneration of tissue.

### The polymers

The biodegradable polymers of the biodegradable, porous materials are composed of a polyalkylene glycol residue and one or two poly(lactide-co-glycolide) residue(s).

Hence, the biodegradable polymers have the general formula:

A-O-(CHR¹CHR²O)ₙB

wherein
A is a poly(lactide-co-glycolide) residue of a molecular weight of at least 4000 g/mol, the molar ratio of (i) lactide units C-CH(CH₃)-COO-] and (ii) glycolide units [-CH₂-COO-] in the poly(lactide-co-glycolide) residue being in the range of 80:20 to 10:90, in particular 70:30 to 10:90,
B is either a poly(lactide-co-glycolide) residue as defined for A or is selected from the group consisting of hydrogen, C₁₋₆-alkyl and hydroxy protecting groups,
one of R¹ and R² within each -(CHR¹CHR²O)- unit is selected from hydrogen and methyl, and the other of R¹ and R² within the same -(CHR¹CHR²O)- unit is hydrogen,
n represents the average number of -(CHR¹CHR²O)- units within a polymer chain and is an integer in the range of 10-1000, in particular 16-250,
the molar ratio of (iii) polyalkylene glycol units [-(CHR¹CHR²O)-] to the combined amount of (I) lactide units [-CH(CH₃)-COO-] and (ii) glycolide units [-CH₂-COO-] in the poly(lactide-co-glycolide) residue(s) is at the most 14:86,
and wherein the number average molecular weight of the copolymer is at least 10,000 g/mol.

Hence, the polymers can either be of the diblock-type or of the triblock-type.

It is understood that the polymer comprises either one or two residues A, i.e. poly(lactide-co-glycolide) residue(s). It is found that such residues should have a molecular weight of at least 4000 g/mol, more particularly at least 5000 g/mol, or even at least 8000 g/mol.

The poly(lactide-co-glycolide) of the polymer can be degraded under physiological conditions, e.g. in bodily fluids and in tissue. However, due to the molecular weight of these residues (and the other requirements set forth herein), it is believed that the degradation will be sufficiently slow so that materials and objects made from the polymer can fulfil their purpose before the polymer is fully degraded.

The expression "poly(lactide-co-glycolide)" encompasses a number of polymer variants, e.g. poly(random-lactide-co-glycolide), poly(DL-lactide-co-glycolide), poly(mesolactide-co-glycolide), poly(L-lactide-co-glycolide), the sequence of lactide/glycolide in the PLGA can be either random, tapered or as blocks and the lactide can be either L-lactide, DL-lactide, D-lactide, or meso-lactide.

Preferably, the poly(lactide-co-glycolide) is a poly(random-lactide-co-glycolide) or poly(tapered-lactide-co-glycolide).

Another important feature is the fact that the molar ratio of (i) lactide units [-CH(CH₃)-COO-] and (ii) glycolide units [-CH₂-COO-] in the poly(lactide-co-glycolide) residue(s) should be in the range of 80:20 to 10:90, in particular 70:30 to 10:90.

It has generally been observed that the best results are obtained for polymers wherein the molar ratio of (i) lactide units [-CH(CH₃)-COO-] and (ii) glycolide units [-CH₂-COO-] in the poly(lactide-co-glycolide) residue(s) is 70:20 or less, or 70:30 or less, however fairly good results were also observed when for polymer having a respective molar ratio of up to 80:20 as long as the molar ratio of (iii) polyalkylene glycol units [-(CHR¹CHR²O)-] to the combined amount of (i) lactide units [-CH(CH₃)-COO-] and (ii) glycolide units [-CH₂-COO-] in the poly(lactide-co-glycolide) residue(s) was at the most 10:90.

As mentioned above, B is either a poly(lactide-co-glycolide) residue as defined for A or is selected from the group consisting of hydrogen, C₁₋₆-alkyl and hydroxy protecting groups.

In one embodiment, B is a poly(lactide-co-glycolide) residue as defined for A, i.e. the polymer is of the triblock-type.

In another embodiment, B is selected from the group consisting of hydrogen, C₁₋₆-alkyl and hydroxy protecting groups, i.e. the polymer is of the diblock-type.

Most typically (within this embodiment), B is C₁₋₆-alkyl, e.g. methyl; ethyl, 1-propyl, 2-propyl, 1-butyl, tert-butyl, 1-pentyl, etc., most preferably methyl. In the event where B is hydrogen, i.e. corresponding to a terminal OH group, the polymer is typically prepared using a hydroxy protecting group as B. "Hydroxy protecting groups" are groups that can be removed after the synthesis of the polymer by e.g. hydrogenolysis, hydrolysis or other suitable means without destroying the polymer, thus leaving a free hydroxyl group on the PEG-part, see, e.g. textbooks describing state in the art procedures such as those described by Greene, T.W. and Wuts, P. G. M. (Protecting Groups in Organic Synthesis, third or later editions). Particularly useful examples hereof are benzyl, tetrahydropyranyl, methoxymethyl, and benzyloxycarbonyl. Such hydroxy protecting groups may be removed in order to obtain a polymer wherein B is hydrogen.

One of R¹ and R² within each -(CHR¹CHR²O)- unit is selected from hydrogen and methyl, and the other of R¹ and R² within the same -(CHR¹CHR²O)- unit is hydrogen. Hence, the -(CHR¹CHR²O)ₙ- residue may either be a polyethylene glycol, a polypropylene glycol, or a poly(ethylene glycol-co-propylene glycol). Preferably, the -(CHR¹CHR²O)ₙ- residue is a polyethylene glycol, i.e. both of R¹ and R² within each unit are hydrogen.

n represents the average number of -(CHR¹CHR²O)- units within a polymer chain and is an integer in the range of 10-1000, in particular 16-250. It should be understood that n represents the average of -(CHRCHR²O)- units within a pool of polymer molecules. This will be obvious for the person skilled in the art. The molecular weight of the polyalkylene glycol residue (CHR¹CHR²O)ₙ-) is typically in the range of 750-10,000 g/mol, e.g. 750-5,000 g/mol.

The -(CHR¹CHR²O)ₙ- residue is typically not degraded under physiological conditions, by may - on the other hand - be secreted unaltered from the human body.

The molar ratio of (iii) polyalkylene glycol units [-(CHR¹CHR²O)-] to the combined amount of (i) lactide units [₋CH(CH₃)-COO-] and (ii) glycolide units [-CH₂-COO-] in the poly(lactide-co-glycolide) residue(s) also plays a certain role and should be at the most 14:86. More typically, the ratio is at the most 12:88, in particular at the most 10:90, or even at the most 8:92. Often, the ratio is in the range of 0.5:99.5 to 14:86, such as in the range of 1:99 to 14:86, or in the range of 1:99 to 12:88, in particular in the range of 2:98 to 10:90, or even in the range of 2:98 to 8:92.

It is believed that the molecular weight of the copolymer for use in the porous.materials is not particularly relevant as long as it is at least 10,000 g/mol. Preferably, however, the molecular weight is at least 15,000 g/mol. The "molecular weight" is to be construed as the number average molecular weight of the polymer, because the skilled person will appreciate that the molecular weight of polymer molecules within a pool of polymer molecules will be represented by values distributed around the average value, e.g. represented by a Gaussian distribution. More typically, the molecular weight is in the range of 10,000-1,000,000 g/mol, such as 15,000-250,000 g/mol, or 20,000-200,000 g/mol. Particularly interesting polymers are found to be those having a molecular weight of at least 20,000 g/mol, such as at least 30,000 g/mol.

The polymer structure may be illustrated as follows (where R is selected from hydrogen, C₁₋₆-alkyl and hydroxy protecting groups; n is as defined above, and m, p and ran are selected so that the above-mentioned provisions for the poly(lactide-co-glycolide) residue(s) are fulfilled): 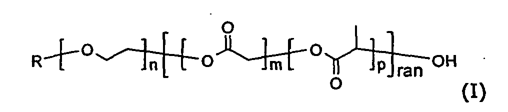 diblock-type polymer 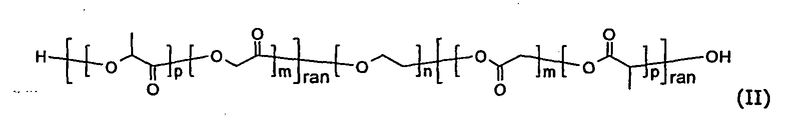 triblock-type polymer

For each of the above-mentioned polymer structures (I) and (II) will be appreciated that the lactide and glycolide units [-CH₂-COO-] represented by p and m may be randomly distributed depending on the starting materials and the reaction conditions.

Also, it is appreciated that the lactide units [-CH(CH₃)-COO-] may be either D/L or L or D, typically D/L or L, or meso-lactide.

As mentioned above, the poly(lactide-co-glycolide) residue(s), i.e. the polyester residue(s), is/are degraded hydrolytically in physiological environments, and the polyalkylene glycol residue is secreted from, e.g., the mammalian body. The biodegradability can be assessed as outlined in the Experimentals section.

### Preparation of polymers

The polymers can in principle be prepared following principles known to the person skilled in the art.

In principle, polymer where B is not a residue A (diblock-type polymers) can be prepared as follows:

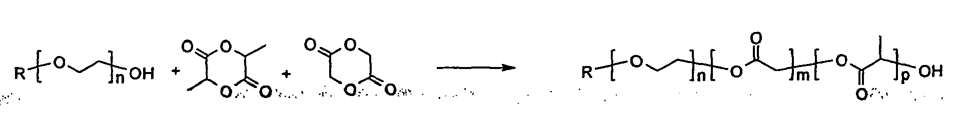

In principle, polymer where B is a residue A (triblock-type polymers) can be prepared as follows:

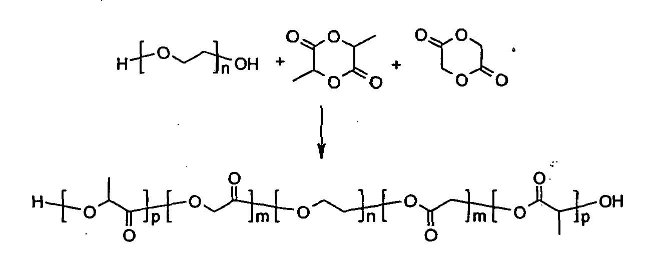

Unless special conditions are applied, the distribution of lactide units [-CH(CH₃)-COO-] and glycolide units [-CH₂-COO-] will be randomly distributed or tapered within each poly(lactic-co-glycolide) residue.

The synthesis of the polymers according to the invention is further illustrated in the Experimentals section.

### The materials

The.polymers are useful for a wide range of materials for medical, dental and surgical use.

The material is a biodegradable, porous material comprising a polymer as defined herein, wherein the porosity is at least 50%, such as in the range of 50-99.5%.

The high degree of porosity can - as will be described further below - be obtained by freeze-drying, i.e. the material is preferably freeze-dried.

The void space of the material of the polymer may be unoccupied so as to allow or even facilitate cell adhesion and/or in-growth for regeneration of tissue. In one embodiment, however, the pores of the material are at least partly occupied by one or more components from the extracellular matrix. Such components may facilitate the cell adhesion and/or in-growth for regeneration of tissue. Examples of components from the extracellular matrix are chondroitin sulfate, hyaluronan, hyaluronic acid, heparin sulfate, heparan sulfate, dermatan sulfate, growth factors, thrombin, fibrin, fibronectin, elastin, collagen, gelatin, and aggrecan.

The components from the extracellular matrix could be added either as particles, which are heterogeneously dispersed, or as a surface coating. The concentration of the components from the extracellular matrix relative to the synthetic polymer is typically in the range of 0.5-15% (w/w), preferably below 10% (w/w). Moreover, the concentration of the components of the extracellular matrix is preferably at the most 0.3% (w/v), e.g. at the most 0.2 (w/v), relative to the volume of the material. In some embodiments, however, the concentration of the components from the extracellular matrix relative to the synthetic polymer may be as high as 80%, such as 40-70%. Within these embodiments, the concentration of the components of the extracellular matrix is preferably 0.1-5% (w/v), e.g. 0.5-4% (w/v).

With respect to biodegradability, it is in some embodiments preferred that the degree of degradation of the material is in the range of 0.9-0.1, such as 0.8-0.2, when tested for 28 days in the Biodegradation Test described in the Experimentals section.

The porous materials may be prepared according to known techniques, e.g. as disclosed in Antonios G.Mikos, Amy J.Thorsen, Lisa A Cherwonka, Yuan Bao & Robert Langer. Preparation and characterization of poly(L-lactide) foams. Polymer 35, 1068-1077 (1994). One very useful technique for the preparation of the porous materials is, however, freeze-drying.

Hence, the present invention also provides a method for the preparation of a biodegradable, porous material of a polymer, the method comprising the steps of:
(a) dissolving a polymer as defined herein in a non-aqueous solvent so as to obtain a polymer solution;
(b) freezing the solution obtained in step (a) so as to obtain a frozen polymer solution; and
(c) freeze-drying the frozen polymer solution obtained in step (b) so as to obtain the biodegradable, porous material.

The non-aqueous solvent used in the method should with respect to melting point be selected so that it can be suitable frozen. Illustrative examples hereof are dioxane (mp. 12°C) and dimethylcarbonate (mp. 4°C).

In one variant, the polymer solution, after step (a) is poured or cast into a suitable mould. In this way, it is possible to obtain a three-dimensional shape of the material specifically designed for the particular application.

Moreover, the invention also encompasses a variant wherein particles of components from the extracellular matrix are dispersed in the solution obtained in step (a) before the solution (dispersion) is frozen at defined in step (b).

The invention also encompasses the more specific variant wherein the components from the extracellular matrix are dissolved in a suitable solvent and then added to the solution obtained in step (a). By mixing with the solvent of step (a), i.e. a solvent for the polymer defined herein, the components from the extracellular matrix will most likely precipitate so as to form a dispersion.

Further, the invention encompasses a variant wherein the biodegradable, porous material obtained in step (c), in a subsequent step, is immersed in a solution of glucosaminoglycan (e.g. hyaluronan) and subsequently freeze-dried again.

The invention also provides a medical device element of a material comprising a polymer as defined herein.

When used in the present context, the expression "medical device" is intended to encompass wound dressings, sutures, implants, etc. The term "element" is intended to mean a certain part, section, or layer of said medical device.

Hence, in one embodiment, the element is the outer coating of a medical device.

In another embodiment, the material forms the outer layer of a wound dressing.

In a particular variant, the invention provides a medical device of a material comprising a polymer as defined herein, i.e. the medical device is made entirely of the material. In one variant, the medical device is in the form of a mesh or net, in particular a mesh, such as a hernia mesh. In another variant, the medical device is in the form of a fibre, in particular a suture.

In some alternative embodiments, the material are present in the form of a fibre or a fibrous structure prepared from the polymer defined herein, possibly in combination with components from the extracellular matrix. Fibres or fibrous materials may be prepared by techniques known to the person skilled in the art, e.g. by melt spinning, electrospinning, extrusion, etc. Such fibres or fibrous materials may, e.g., be used as sutures, hernia-meshes, scaffolds, etc.

### Various uses

As it will be obvious from the above, the polymers and materials have a multitude of uses within the field of medicine, healthcare, surgery, dentistry, etc., in particular uses where a biodegradable polymer is required, e.g. for wound dressings, scaffolds for cell attachment and in-growth for regeneration of tissue, sutures, hernia-mesh, cartilage, ligaments, implants, etc.

Hence, the present invention also relates to a biodegradable, porous material as defined herein for use in therapy, dentistry or surgery.

More particular, the invention also relates to the use of a biodegradable, porous material as defined herein for the preparation of a scaffold for supporting cell adhesion or the in-growth for regeneration of tissue, and to the use of a biodegradable, porous material as defined herein for the preparation of a wound dressing.

### EXPERIMENTALS

### Biodegradation Test

Biodegradability of the porous material can be determined as follows.

Approx. 1 gram of a porous material is fully immersed in a medium (10% foetal calf serum in DMEM (Dulbecco's modified Eagle's medium)) at is store at 37°C for a period of 28 days. The medium is changed twice a week, i.e. on days 3, 7, 10, 14, 17, 21, and 24. On day 28, the porous material is analysed by GCP. The biodegradation is measured as the number/weight average molecular weight relative to the initial value.

A porous MPEG-PLGA (2-30 kDa, L:G 50:50) was tested, and the biodegradation was determined as approx. 0.5 (final M_{n/w} value relative to initial value)

### Summary

As an attempt to make PLGA more hydrophilic, MPEG or PEG was copolymerised with PLGA to give copolymers with a low MPEG/PEG-content (<20% (w/w) MPEG/PEG). When these polymers were tested and compared to plain PLGA, the initial adhesions of cells to MPEG-PLGA and PLGA-PEG-PLGA were superior to plain PLGA and the morphology and attachment of the cells were better.

This is surprising, since PEG-containing polymers are known from the literature to resist the adhesion of proteins and cells. The key to the improved performance of our polymers seems to be that the PEG-content in the polymer is kept low (at the most 14 mol-%) as polymers with high PEG-content gave poor adhesion and morphology in the biological tests.

PLA have long degradation-times compared to PLGA, and our experiments show that a higher lactide content in the PLGA-part of polyether-PLGA give a slower adhesion of cells.

Known synthetic biodegradable polymers are typically hydrophobic materials with sluggish initial cell adhesion in a biological environment. We attempted to modify the hydrophilicity of PLGA by synthesizing an MPEG-PLGA block copolymer. Our first polymer was a 1.9-30 kDa MPEG-PLGA with an G:L-ratio of 50:50 (mol). These are made into thin porous sheets by freeze-drying. In a biological assay both the initial and long-term cell adhesion was excellent, and the performance was superior to the unmodified PLGA. This is surprising since the literature describes that incorporation of PEG into polymers make them resistant to the adhesion of cells and proteins. The key to our success seems to be that we have a low PEG-content (6% (w/w)) (see Table 2 below). When the PEG content is higher (MPEG-PLGA 5-30 kDa, 14% (w/w) PEG) we see a reduced cell adhesion, both initial and longer term when compared to the low-PEG materials and plain PLGA.

### Purification of reagents

DL-lactide and glycolide are recrystallized in dry ethylacetate in a nitrogen atmosphere and dried with vacuum. PEG/MPEG is dissolved in a suitable solvent, precipitated in cold hexane, filtered, and dried overnight. Stannous 2-ethylhexanoate is vacuum-distilled and stored under nitrogen. Ethyl acetate is distilled from calcium hydride under nitrogen. Dioxane is distilled from sodium/benzophenone under nitrogen. Toluene is distilled from sodium/benzophenone under nitrogen.

### Synthesis of polymers

PEG/MPEG, DL-lactide, glycolide and 4%(w/v) stannous octanoate in toluene are added to a vial in a glove box with nitrogen atmosphere. The vial is closed, heated and shaken until the contents are clear and homogeneous and then placed in an oven at 120-200°C for 1 min to 48 hours, e.g. up to 6 h.

The synthesis can also be made in a solution in a suitable solvent (e.g. dioxane) to facilitate the subsequent purification. Then MPEG, DL-lactide, glycolide, 4% stannous 2-ethylhexanoate and 100% (w/w) dioxane are added to a vial in a glove box with nitrogen atmosphere, and treated as above.

### Purification of polymer

The polymer (see Table 1) is dissolved in a suitable solvent (e.g. dioxane, tetrahydrofuran, chloroform, acetone), and precipitated with stirring in a non-solvent (e.g. water, methanol, ethanol, 1-propanol or 2-propanol) at a temperature of -40 to 40°C. The polymer is left to settle, solvent discarded and the polymer is dried in a vacuum oven at 40-120°C/ovemight.

The polymers are analyzed with NMR-spectroscopy and GPC to confirm structure, molecular weight and purity.

**Table 1 - Examples of the synthesis of various MPEG-PLGA polymers**

| Polymer | G/L-ratio | glycolide (g) | DL-lactide (g) | Initiator | initiator (g) | 4% Sn(Oct)₂ (µL) | dioxane (g) |
|---|---|---|---|---|---|---|---|
| 750-15000 50L | 50:50 | 2.12 | 2.64 | MPEG 750 Da | 0.238 | 129 | 5 |
| 1100-20000 50L | 50:50 | 2.11 | 2.63 | MPEG 1100 Da | 0.261 | 98 | 5 |
| 1900-30000 50L | 50:50 | 2.10 | 2.60 | MPEG 1900 Da | 0.298 | 65 | 5 |
| 1900-30000 80L | 20:80 | 0.79 | 3.91 | MPEG 1900 Da | 0.298 | 65 | 5 |
| 5000-30000 50L | 50:50 | 1.86 | 2.31 | MPEG 5000 Da | 0.833 | 68 | 5 |
| 15000-1900-15000 50L | 50:50 | 2.10 | 2.60 | PEG 1900 Da | 0.298 | 65 | 5 |
| 30000-5000-30000 50L | 50:50 | 2.06 | 2.56 | PEG 5000 Da | 0.385 | 31 | 5 |

### Process for making scaffolds

Polymer (e.g. 1.9-30 kDa) is dissolved in a suitable solvent (e.g. dioxane) to a concentration of 0.5-10% (w/v). The solution is poured in a mold, frozen and freeze-dried to a porous sheet. Components from the extracelluar matrix may be incorporated either by dispersing such components in the solvent or by subsequently treating the porous sheet with a dispersion/solution of components from the extracellular matrix.

### Testing of scaffolds

Biocompatibility studies (see Table 2) of the different scaffolds of MPEG-PLGA and PLGA were performed by seeding primary fibroblast at a concentration of 2.5 x 10⁴ cells/cm² on the surface of the scaffolds. Evaluation of the cells attachment, viability and growth were performed at day 1, 3 and 7 by staining the cells using neutral red followed by evaluation using an Leica DMIRE2 inverted microscope fitted with a Evolution MP cooled color camera (Media Cybernetics) and digital images were taken using Image Pro Plus 5.1 software (Media Cybernetics).

Studies comparing the biocompatibility of freeze dried scaffolds of PLGA showed generally adhering of cells with fine morphology but very low initial amount of cells. Comparing these scaffolds with MPEG-PLGA 2-30 kDa, we see a better biocompatibility of the MPEG-PLGA scaffold because of a higher amount of cells are adhering to this scaffold due to a better wetting ability.

Cells are growing with a fine morphology and good adherence to MPEG-PLGA 1.9-30 kDa from the start of the test and an increase in amount of cells are seen from day 1 to day 7. Increasing the size of the MPEG part of the MPEG-PLGA to 5-30 kDa gives solely rounded cells with little or no adherence to the surface of the scaffolds giving a pronounced decreased biocompatibility and which is worsened from day 1 to day 7.

If MPEG-PLGA 2-15 kDa is tested and compared with MPEG-PLGA 2-20 kDa and MPEG-PLGA 2-30 kDa, we see an increasing attachment and viability of the fibroblasts when the size of the PLGA part was increased. This means that the 2-30 kDa had the best biocompatibility. Increasing the size from 2-15 kDa to 2-20 kDa gives the largest positive effect on the biocompatibility compared with the step from 2-20 kDa to 2-30 kDa.

Increasing the L:G ratio in MPEG-PLGA 2-20 kDa from 50:50 to 80:20 gives decreased attachment and viability of the fibroblasts.

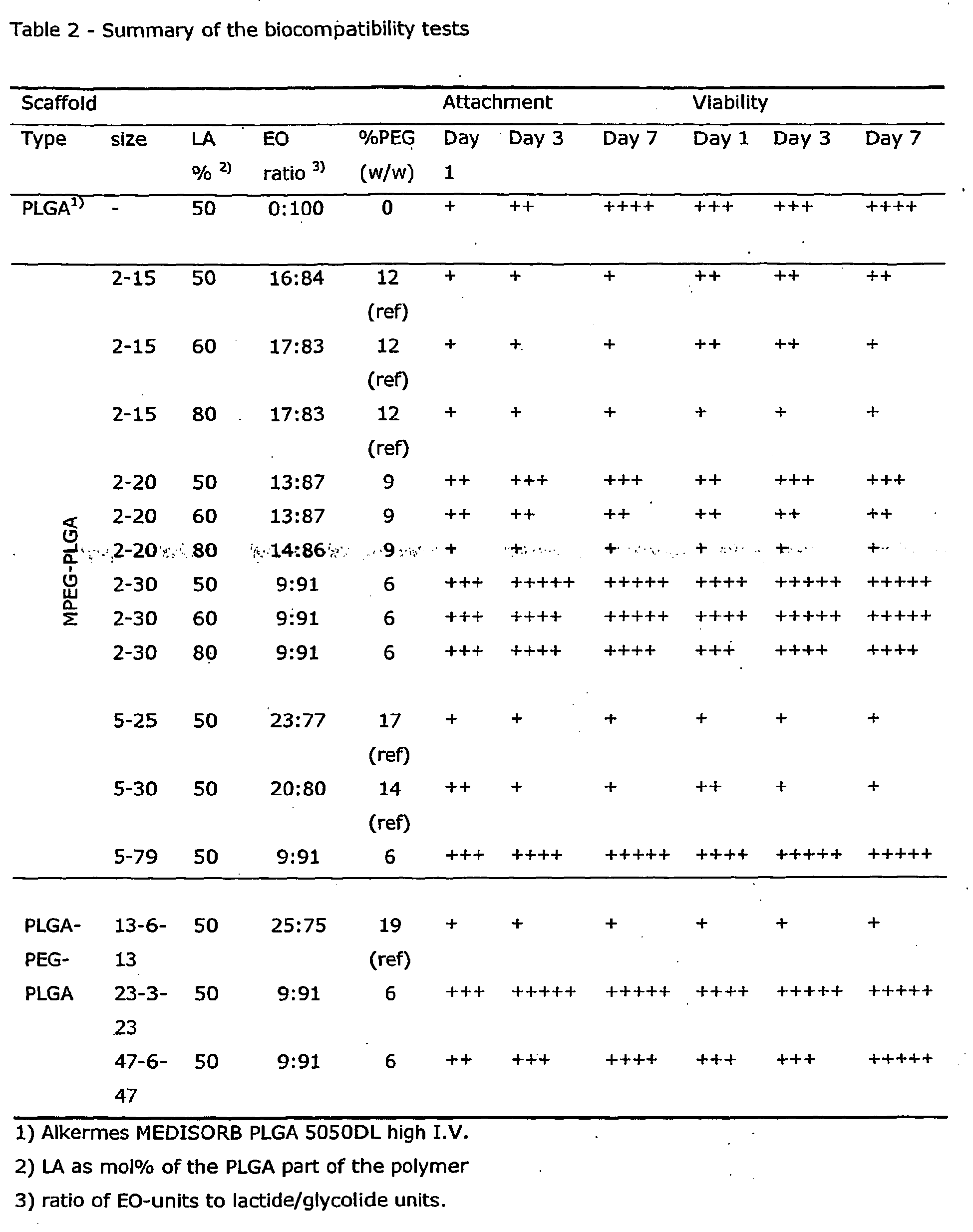

The results are graded subjective from + to +++++ with + meaning low attachment and low viability while +++++ are excellent attachment and viability.

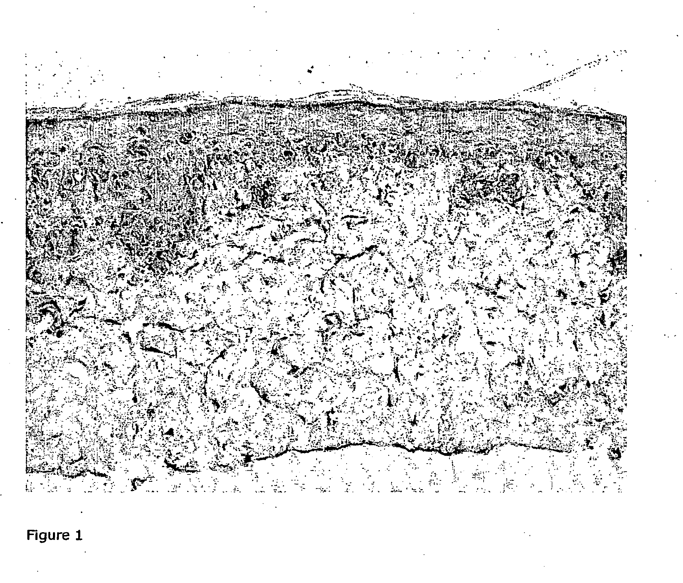

Human keratinocytes can be cultured in vitro on fibroblast populated MPEG-PLGA scaffolds to form a multilayered and differentiated reconstituted epidermis (see Figure 1). The reconstituted epidermis shows morphological features resembling normal epidermis in vivo.

On histological specimens, we find clear evidence of basal cell layers (stratum basale) and ultimately overlying stratum corneum with intervening layers resembling, however, immature and slightly hyperproliferative, spinous and granular layers. Lack of final maturation should be ascribed to the chosen in vitro model rather than the scaffold material.

### Preparation of MEPG-PLGA scaffolds containing ECM particles

Preparation of composite scaffolds of MPEG-PLGA and ECM: Methoxy-polyethylene glycol-poly(lactide-co-glycolide) (Mn 2,000-30,00, L:G 1:1; prepared as described hereinabove) was dissolved in 1,4-dioxane to a 1.5% solution. For UBM (Acell Inc., USA) containing samples, the UBM was added to the solution while stirring; 0, 0.017, 0.038, 0.064, 0.1, 0.15, 0.225 g/scaffold (0, 10, 20, 30, 40, 50, 60% w/w), high-speed-mixed and 10 mL poured in 7.3x7.3 cm mould. The solution was frozen at -5°C and lyophilized at -20°C for 5h and 20°C for approx 15 h. The samples were placed in a desiccator with vacuum overnight to remove residual dioxane.

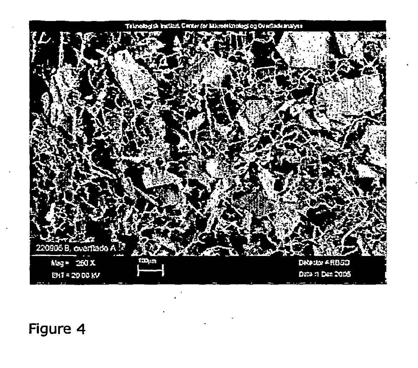

SEM pictures demonstrate that the ECM particles are homogenously distributed in the scaffold matrix Figure 4.

### Preparation, cell morphology and 3D growth in composite scaffolds of MPEG-PLGA and GAG holding 4 different concentrations of GAG.

Preparation of composite scaffolds of MPEG-PLGA and glycosaminoglycans (GAG): Methoxy-polyethylene glycol-poly(lactide-co-glycolide) (Mn 2,000-30,000, L:G 1:1; prepared as described hereinabove) was dissolved in 1,4-dioxane to a 1.5% solution. For GAG containing samples, the GAG was added to the solution while stirring; 0, 0.0015, 0.0031, 0.00625, 0.013 g/scaffold (0, 1, 2, 4, 8% w/w), high-speed-mixed and 10 mL poured in 7.3x7.3 cm mould. The solution was frozen at -5°C and lyophilized at -20°C for 5h and 20°C for approx 15h. The samples were placed in a desiccator with vacuum overnight to remove residual dioxane.

In order to evaluate the cell morphology and 3D growth of the composite scaffolds, biopsies were punched out of each type of the scaffolds and seeded with primary human fibroblasts (passage 3) on the surface of the scaffolds with a density of 2.5 x 10⁴ cells/cm² in a small volume of growth medium (10% FCS. in DMEM) containing antibiotics (penicillin, streptomycin and Amphotericin B). The scaffolds were incubated at 37°C at 5% CO₂ before additional growth medium was added. Evaluation of the cells attachment, morphology, growth and population of the scaffold were preformed on day 1, 3 and 7 by staining the cells with neutral red followed by evaluation using an Leica DMIRE2 inverted microscope fitted with a Evolution MP cooled colour camera (Media Cybernetics). Digital images were taken using Image Pro Plus 5.1 software (Media Cybernetics).

The cell morphology and 3D growth in the MPEG-PLGA scaffold without GAG showed in the first days of the study adherent cells growing as a combination of rounded cells and spindle-shaped cells. The cells were growing on the surface of the scaffolds. During the rest of the study all cells became spindle-shaped and from day 3 observed growing into the scaffolds. Adding 1% (w/w) GAG into the scaffolds did not change the way the cells were growing in this study compared to MPEG-PLGA scaffold without GAG. Increasing the concentration of GAG to 2%(w/w) resulted at day 1 in cells growing more in small region where the cells were growing close together. Increasing the concentration of GAG to 4 and 8% (w/w) increased the sizes of the regions and made it difficult to distinguish separate cells.

At day 3 the cells were beginning to spread more on the surface of the scaffolds. This effect were more pronounced in 2 and 4% (w/w) compared to the scaffolds containing 8% (w/w).

Cells were at day 7 growing more on the surface of the scaffolds in the pure MPEG-PLGA scaffold and low concentration of GAG compared to more in-growth into the scaffolds containing higher concentrations of GAG and as a consequence of this cells were growing more spread with increasing concentrations of GAG.

### GAG release from composite scaffolds of MPEG-PLGA and GAG holding different concentrations of GAG.

Preparation of composite scaffolds of MPEG-PLGA and GAG: Methoxy-polyethylene glycol-poly(lactide-co-glycolide) (Mn 2,000-30,000, L:G 1:1; prepared as described hereinabove) was dissolved in 1,4-dioxane to a 1.5% solution. For GAG containing samples, the GAG was added to the solution while stirring; 0, 0.0015, 0.0031, 0.013 g/scaffold (0, 1, 2, 8% w/w), high-speed-mixed and 10 mL poured in 7.3x7.3 cm mould. The solution was frozen at -5°C and lyophilized at -20°C for 5 h and 20°C for approx 15 h. The samples were placed in a desiccator with vacuum overnight to remove residual dioxane.

In the dimethylmethylene blue (DMMB) assay the sulphated GAG content is measured by an increasing in OD 525nm. The DMMB colour solution was prepared according to Farndale et al. (RW Farndale et al. Biochimica et Biophysica Acta 883:173-177, 1986). Briefly, 16 mg 1,9-dimethylmethylene blue was dissolved in 1 L of water containing 3.04 g glycine, 2.37 g NaCl and 95 mL 0.1 M HCl, pH 3.0. In order to measure the release of GAG from the MPEG-PLGA scaffolds containing 0, 1, 2 and 8% (w/w) GAG biopsies were punched out of each type of the scaffolds. These biopsies in duplicates were placed in a 48 well plate and 200 µl of DMMB solution were poured over the scaffolds corresponding to the amount necessary to cover the scaffolds. Five minutes later 100µl of the colour solution from the wells were transferred to a 96 well plate and measured at 525 nm. A Synergy^{™} HT Multi-Detection Microplate Reader from Bio-Tek was used.

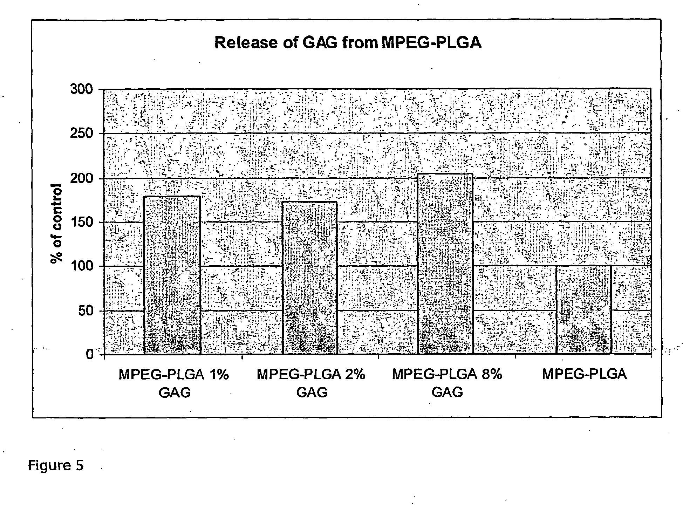

The result of the study showed an immediately release of GAG from all the scaffolds containing GAG and no release from the pure MPEG-PLGA scaffold (see Figure 5). A slightly higher release was observed in the 8% (w/w) scaffold compared to the 1 and 2% (w/w).

### Implantation of Integra and MPEG-PLGA in a 28-day mouse model.

Two groups of 4 mice (NMRI mice from M&B-Taconic) at Pipeline Biotech A/S had an 8 mm biopsy of respectively Integra (Integra LifeSciences Corporation, USA) or MPEG-PLGA scaffolds (1.5% 2-30 50L) implanted under the skin in the neck region. The skin opening was closed with sutures. At day 10 and 28, 2 mice from each group were euthanized with CO₂ and the area of the implantation including the surrounding area was excised and transferred to Lilly's formalin buffer before embedding in paraffin. Each paraffin block was carefully sectioned by 5 µm increments until each scaffold was located and the subsequent slices stained with haematoxylin and Eosin (H&E). Digital images were collected using a BX-60 Olympus light microscope fitted with an Evolution MP cooled colour camera (Media Cybernetics) and digital image were taken using Image Pro Plus 5.1 software. An independent pathologist performed the histopathological evaluation of the study.

The Integra showed a nice integration into the surrounding tissue and a slight fibrotic in-growth along the border of the scaffold at day 10. No fibroblasts were observed in the middle of the scaffolds. Minimal inflammation in scaffolds and surroundings, however, some giant cells were present at day 10. The scaffolds were still intact after 28 days, but without further in-growth of fibroblasts.

MPEG-PLGA showed a very nice integration into the surrounding tissue at day 10, with a mild inflammatory response and significant in-growth of fibroblast cells along the border, corresponding to a desirable fibrotic response, and migrating cells in the middle of the scaffold. In contrast to Integra, the in-growth continued at day 28 with formation of neodermis and without an inflammatory response.

In conclusion, the animal study showed that MPEG-PLGA was the best-suited scaffold material, supportive of fibroblast in-growth, formation of neodermis and well tolerated by the host.

### Cartilage regeneration with chondrocytes in MPEG-PLGA scaffold (in vivo study in goats)

The present study aims to investigate the cartilage regenerative response of a MPEG-PLGA porous scaffold combined with chondrocyte suspension in a goat femoral condyle full thickness cartilage defect model.

### Methods:

10 adult goats were used for the study and the study conducted at the Research Center at Foulum, Denmark. A 6 mm circular defect was created in both medial femoral condyles. Cartilage tissue was harvested for chondrocyte culture. At secondary open surgery the defects were randomized to the following two treatment groups.
1. Empty defect (control)
2. Fibrin/chondrocyte solution in a MPEG-PLGA freeze-dried porous scaffold (4% 2-30 50L).

Animals were followed for 4 month. Analyses: ICRS macroscopic scoring (0-12).

Mechanical test was performed to assess stiffness of regeneration tissue. Histological analyses was performed by O,Driscoll and Pinada cartilage scores and percentage filling of the defects.

### Results:

The ICRS and histology scores demonstrated highly significant difference between groups. The cartilage regeneration in the MPEG-PLGA/Cell group demonstrated high defect fill and a tissue characteristic close to hyaline cartilage whereas no regeneration tissue was seen in the empty defects. Mechanical testing demonstrated no difference between treatment groups.

### Conclusion:

The MPEG-PLGA/cell construct demonstrates an extensive cartilage regenerative response with good phenotypic characteristic. As expected no regeneration was seen in the empty defects. Porous MPEG-PLGA scaffold in combination with cultured chondrocytes seem to be a good technique for cartilage tissue engineering in vivo.

## Claims

1. A biodegradable, porous material comprising a polymer of the general formula
A-O-(CHR¹CHR²O)ₙ-B
wherein
A is a poly(lactide-co-glycolide) residue of a molecular weight of at least 4000 g/mol, the molar ratio of (i) lactide units [-CH(CH₃)-COO-] and (ii) glycolide units [-CH₂-COO-] in the poly(lactide-co-glycolide) residue being in the range of 80:20 to 10:90,
B is either a poly(lactide-co-glycolide) residue as defined for A or is selected from the group consisting of hydrogen, C₁₋₆-alkyl and hydroxy protecting groups,
one of R¹ and R² within each -(CHR¹CHR²O)- unit is selected from hydrogen and methyl, and the other of R¹ and R² within the same -(CHR¹CHR²O)- unit is hydrogen,
n represents the average number of -(CHR¹CHR²O)- units within a polymer chain and is an integer in the range of 10-1000,
the molar ratio of (iii) polyalkylene glycol units [-(CHR¹CHR²O)-] to the combined amount of (i) lactide units [-CH(CH₃)-COO-] and (ii) glycolide units [-CH₂-COO-] in the poly(lactide-co-glycolide) residue(s) is at the most 14:86,
and wherein the number average molecular weight of the copolymer is at least 10,000 g/mol.
wherein the porosity is at least 50%.

2. The porous material according to claim 1, wherein the number average molecular weight of the copolymer is at least 20,000 g/mol.

3. The porous material according to any one of the preceding claims, wherein both of R¹ and R² within each unit are hydrogen.

4. The porous material according to any one of the preceding claims, wherein B is a poly(lactide-co-glycolide) residue as defined for A.

5. The porous material according to any one of the preceding claims, wherein B is C₁₋₆-alkyl.

6. The porous material according to any one of the preceding claims, wherein B is a hydroxy protecting group.

7. The porous material according to any one of the preceding claims, wherein B is hydrogen.

8. The porous material according to any one of the preceding claims, wherein the polymer has the general formula:
A-O-(CH₂CH₂O)ₙ-methyl
wherein
A is a poly(lactide-co-glycolide) residue of a molecular weight of at least 8,000 g/mol, the molar ratio of (i) lactide units [-CH(CH₃)-COO-] and (ii) glycolide units [-CH₂-COO-] in the poly(lactide-co-glycolide) residue being in the range of 80:20 to 10:90,
n represents the average number of -(CH₂CH₂O)- units within a polymer chain and is an integer in the range of 16-250,
the molar ratio of (iii) polyethylene glycol units [-(CH₂CH₂O)-] to the combined amount of (i) lactide units [-CH(CH₃)-COO-] and (ii) glycolide units [-CH₂-COO-] in the poly(lactide-co-glycolide) residue(s) is in the range of 1:99 to 12:88,
and wherein the number average molecular weight of the copolymer is at least 20,000 g/mol.

9. The material according to any one of the claims 1-8, which is freeze-dried.

10. The material according to any one of the claims 1-9, wherein the pores of the material is at least partly occupied by one or more components from the extracellular matrix.

11. The material according to claim 10, wherein the component from the extracellular matrix is selected from chondroitin sulfate, hyaluronan, hyaluronic acid, heparin sulfate, heparan sulfate, dermatan sulfate, growth factors, thrombin, fibrin, fibronectin, elastin, collagen, gelatin, and aggrecan.

12. A biodegradable polymer of the general formula:
A-O-(CH₂CH₂O)ₙ-methyl
wherein
A is a poly(lactide-co-glycolide) residue of a molecular weight of at least 8,000 g/mol, the molar ratio of (i) lactide units [-CH(CH₃)-COO-] and (ii) glycolide units [-CH₂-COO-] in the poly(lactide-co-glycolide) residue being in the range of 80:20 to 10:90,
n represents the average number of -(CH₂CH₂O)- units within a polymer chain and is an integer in the range of 16-250,
the molar ratio of (iii) polyethylene glycol units [-(CH₂CH₂O)-] to the combined amount of (i) lactide units [-CH(CH₃)-COO-] and (ii) glycolide units [-CH₂-COO-] in the poly(lactide-co-glycolide) residue(s) is in the range of 1:99 to 12:88,
and wherein the number average molecular weight of the copolymer is at least 20,000 g/mol.

13. A method for the preparation of a biodegradable, porous material of a polymer, the method comprising the steps of:
(a) dissolving a polymer as defined in any one of the claims 1-8 in a non-aqueous solvent so as to obtain a polymer solution;
(b) freezing the solution obtained in step (a) so as to obtain a frozen polymer solution; and
(c) freeze-drying the frozen polymer solution obtained in step (b) so as to obtain the biodegradable, porous material.

14. The method according to claim 13, wherein the polymer solution, after step (a) is poured or cast into a suitable mould.

15. A medical device element of a material comprising a polymer as defined in claim 12.

16. The medical device element according to claim 15, wherein the element is the outer coating of a medical device.

17. A medical device of a material comprising a polymer as defined in claim 12.

18. The medical device according to claim 17, which is in the form of a mesh or net.

19. The medical device according to claim 17, which is in the form of a fibre.

20. A biodegradable, porous material as defined in any one of the claims 1-11 for use in therapy, dentistry or surgery.

21. The use of a biodegradable, porous material as defined in any one of the claims 1-11 for the preparation of a scaffold for supporting cell adhesion or the in-growth for regeneration of tissue.

22. The use of a biodegradable, porous material as defined in any one of the claims 1-11 for the preparation of a wound dressing.

## Patentansprüche

1. Biologisch abbaubares, poröses Material, das ein Polymer der allgemeinen Formel
A-O-(CHR¹CHR²O)ₙ-B
umfasst,
in der bedeuten:
A einen Poly(lactid-co-glycolid)-Rest mit einem Molekulargewicht von mindestens 4000 g/mol, wobei das Molverhältnis von (i) Lactid-Einheiten [-CH(CH₃)-COO-] zu (ii) Glycolid-Einheiten [-CH₂-COO-] in dem Poly(lactid-co-glycolid)-Rest im Bereich von 80 : 20 bis 10 : 90 liegt,
B entweder einen Poly(lactid-co-glycolid)-Rest wie für A definiert oder einen Rest, der aus der Gruppe ausgewählt ist, die aus Wasserstoff, C₁₋₆-Alkyl und Hydroxy-Schutzgruppen besteht, einer der Substituenten R¹ und R² in jeder Einheit
-(CHR¹CHR²O)- Wasserstoff oder Methyl und der andere der Substituenten R¹ und R² in der gleichen Einheit -(CHR¹CHR²O)-Wasserstoff und
n die mittlere Anzahl von Einheiten -(CHR¹CHR²O)- innerhalb einer Polymerkette und eine ganze Zahl im Bereich von 10 bis 1000,
wobei das Molverhältnis von (iii) Polyalkylenglycol-Einheiten [-(CHR¹CHR²O)-] zur Gesamtmenge von (i) Lactid-Einheiten [-CH(CH₃)-COO-] und (ii) Glycolid-Einheiten [-CH₂-COO-] in dem Poly(lactid-co-glycolid)-Rest oder in den Poly(lactid-co-glycolid)-Resten höchstens 14 : 86 beträgt,
das Zahlenmittel des Molekulargewichts des Copolymers mindestens gleich 10 000 g/mol ist
und die Porosität mindestens 50 % beträgt.

2. Poröses Material nach Anspruch 1, bei dem das Zahlenmittel des Molekulargewichts des Copolymers mindestens 20 000 g/mol beträgt.

3. Poröses Material nach einem der vorhergehenden Ansprüche, bei dem R¹ und R² innerhalb jeder Einheit Wasserstoff bedeuten.

4. Poröses Material nach einem der vorhergehenden Ansprüche, bei dem B einen Poly(lactid-co-glycolid)-Rest bedeutet, wie er für A definiert ist.

5. Poröses Material nach einem der vorhergehenden Ansprüche, bei dem B C₁₋₆-Alkyl bedeutet.

6. Poröses Material nach einem der vorhergehenden Ansprüche, bei dem B eine Hydroxy-Schutzgruppe bedeutet.

7. Poröses Material nach einem der vorhergehenden Ansprüche, bei dem B Wasserstoff bedeutet.

8. Poröses Material nach einem der vorhergehenden Ansprüche, bei dem das Polymer die allgemeine Formel
A-O-(CH₂CH₂O)ₙ-Methyl
besitzt,
worin bedeuten:
A einen Poly(lactid-co-glycolid)-Rest mit einem Molekulargewicht von mindestens 8000 g/mol, wobei das Molverhältnis von (i) Lactid-Einheiten [-CH(CH₃)-COO-] und (ii) Glycolid-Einheiten [-CH₂-COO-] in dem Poly(lactid-co-glycolid)-Rest im Bereich von 80 : 20 bis 10 : 90 liegt, und
n die mittlere Anzahl von Einheiten -(CH₂CH₂O)- innerhalb einer Polymerkette und eine ganze Zahl im Bereich von 16 bis 250, wobei das Molverhältnis von (iii) Polyalkylenglycol-Einheiten [-(CH₂CH₂O)-] zur Gesamtmenge von (i) Lactid-Einheiten [-CH(CH₃)-COO-] und (ii) Glycolid-Einheiten [-CH₂-COO-] in dem Poly(lactid-co-glycolid)-Rest oder in den Poly(lactid-co-glycolid)-Resten im Bereich von 1 : 99 bis 12 : 88 liegt
und das Zahlenmittel des Molekulargewichts des Copolymers mindestens gleich 20 000 g/mol ist.

9. Material nach einem der Ansprüche 1 bis 8, das gefriergetrocknet ist.

10. Material nach einem der Ansprüche 1 bis 9, bei dem die Poren des Materials mindestens zum Teil von einem oder mehreren Bestandteilen aus der extrazellulären Matrix eingenommen werden.

11. Material nach Anspruch 10, bei dem der Bestandteil aus der extrazellulären Matrix ausgewählt ist unter Chondroitinsulfat, Hyaluronan, Hyaluronsäure, Heparinsulfat, Heparansulfat, Dermatansulfat, Wachstumsfaktoren, Thrombin, Fibrin, Fibronectin, Elastin, Collagen, Gelatine und Aggrecan.

12. Biologisch abbaubares Polymer der allgemeinen Formel
A-O-(CH₂CH₂O)ₙ-Methyl,
in der bedeuten:
A einen Poly(lactid-co-glycolid)-Rest mit einem Molekulargewicht von mindestens 8000 g/mol, wobei das Molverhältnis von (i) Lactid-Einheiten [-CH(CH₃)-COO-] und (ii) Glycolid-Einheiten [-CH₂-COO-] in dem Poly(lactid-co-glycolid)-Rest im Bereich von 80 : 20 bis 10 : 90 liegt, und
n die mittlere Anzahl von Einheiten [-(CH₂CH₂O)-] innerhalb einer Polymerkette und eine ganze Zahl im Bereich von 16 bis 250, wobei das Molverhältnis von (iii) Polyethylenglycol-Einheiten [-(CH₂CH₂O)-] zur Gesamtmenge von (i) Lactid-Einheiten [-CH(CH₃)-COO-] und (ii) Glycolid-Einheiten [-CH₂-COO-] in dem Poly(lactid-co-glycolid)-Rest oder in den Poly(lactid-co-glycolid)-Resten im Bereich von 1 : 99 bis 12 : 88 liegt
und das Zahlenmittel des Molekulargewichts des Copolymers mindestens 20 000 g/mol beträgt.

13. Verfahren zur Herstellung eines biologisch abbaubaren, porösen Materials aus einem Polymer, wobei das Verfahren folgende Schritte umfasst:
(a) Lösen eines Polymers, wie es in einem der Ansprüche 1 bis 8 definiert ist, in einem nichtwässerigen Lösungsmittel unter Erhalt einer Polymerlösung,
(b) Einfrieren der in Schritt (a) erhaltenen Lösung unter Erhalt einer gefrorenen Polymerlösung und
(c) Gefriertrocknen der in Schritt (b) erhaltenen gefrorenen Polymerlösung unter Erhalt des biologisch abbaubaren, porösen Materials.

14. Verfahren nach Anspruch 13, bei dem die Polymerlösung nach Schritt (a) in eine geeignete Form eingegossen oder darin einem Formguss unterworfen wird.

15. Element einer medizinischen Vorrichtung aus einem Material, das ein Polymer umfasst, wie es in Anspruch 12 definiert ist.

16. Element einer medizinischen Vorrichtung nach Anspruch 15, wobei es sich bei dem Element um die äußere Beschichtung einer medizinischen Vorrichtung handelt.

17. Medizinische Vorrichtung aus einem Material, das ein Polymer umfasst, wie es in Anspruch 12 definiert ist.

18. Medizinische Vorrichtung nach Anspruch 17, die in Form eines Siebes oder eines Netzes vorliegt.

19. Medizinische Vorrichtung nach Anspruch 17, die in Form einer Faser vorliegt.

20. Biologisch abbaubares, poröses Material wie in einem der Ansprüche 1 bis 11 definiert zur Verwendung bei der Therapie, in der Zahnmedizin oder in der Chirurgie.

21. Verwendung eines biologisch abbaubaren, porösen Materials wie in einem der Ansprüche 1 bis 11 definiert zur Herstellung eines Gerüstes als Träger für eine Anheftung von Zellen oder zum Einwachsen zur Regeneration von Geweben.

22. Verwendung eines biologisch abbaubaren, porösen Materials wie in einem der Ansprüche 1 bis 11 definiert zur Herstellung eines Wundverbands.

## Revendications

1. Matériau poreux biodégradable comprenant un polymère dont la formule générale est
A-O-(CHR¹CHR²O)ₙ-B
dans lequel
A représente un résidu de poly(lactide-co-glycolide) présentant un poids moléculaire d'au moins 4000 g/mol, le rapport molaire des (i) unités de lactide [-CH(CH₃)-COO-] et des (ii) unités de glycolide [-CH₂-COO-] dans le résidu de poly(lactide-co-glycolide) étant compris entre 80:20 et 10:90,
B représente un résidu de poly(lactide-co-glycolide) tel que défini pour A ou est sélectionné dans le groupe comprenant l'hydrogène, l'alkyle C₁₋₆ et les groupes protecteurs hydroxy,
l'un des éléments parmi R¹ et R² dans chaque unité -(CHR¹CHR²O)-est sélectionné parmi l'hydrogène et le méthyle et l'autre élément parmi R¹ et R² dans la même unité -(CHR¹CHR²O)- est de l'hydrogène,
n représente le nombre moyen d'unités -(CHR¹CHR²O)- dans une chaîne polymère et est un nombre entier compris entre 10-1000,
le rapport molaire des (iii) unités de polyalkylène-glycol [-(CHR¹CHR²O)-] par rapport à la quantité combinée des (i) unités de lactide [-CH(CH₃)-COO-] et des (ii) unités de glycolide [-CH₂-COO-] dans le(s) résidu(s) de poly(lactide-co-glycolide) est au maximum de 14:86,
et dans lequel le poids moléculaire moyen en nombre du copolymère est d'au moins 10000 g/mol,
dans lequel la porosité est d'au moins 50 %.

2. Le matériau poreux selon la revendication 1, dans lequel le poids moléculaire moyen en nombre du copolymère est d'au moins 20000 g/mol.

3. Le matériau poreux selon l'une quelconque des revendications précédentes, dans lequel R¹ et R² dans chaque unité sont tous deux de l'hydrogène.

4. Le matériau poreux selon l'une quelconque des revendications précédentes, dans lequel B est un résidu de poly(lactide-co-glycolide) tel que défini pour A.

5. Le matériau poreux selon l'une quelconque des revendications précédentes, dans lequel B est de l'alkyle C₁₋₆.

6. Le matériau poreux selon l'une quelconque des revendications précédentes, dans lequel B est un groupe protecteur hydroxy.

7. Le matériau poreux selon l'une quelconque des revendications précédentes, dans lequel B est de l'hydrogène.

8. Matériau poreux selon l'une quelconque des revendications précédentes, dans lequel le polymère présente la formule générale :
A-O-(CH₂CH₂O)ₙ-méthyl
dans lequel
A est un résidu de poly(lactide-co-glycolide) ayant un poids moléculaire d'au moins 8000 g/mol, le rapport molaire des (i) unités de lactide [-CH(CH₃)-COO-] et des (ii) unités de glycolide [-CH₂-COO-] dans le résidu de poly(lactide-co-glycolide) étant compris entre 80:20 et 10:90,
n représente le nombre moyen d'unités -(CH₂CH₂O)- dans une chaîne polymère et est un nombre entier compris entre 16-250,
le rapport molaire des (iii) unités de polyéthylène-glycol [-CH₂CH₂O)-] par rapport à la quantité combinée des (i) unités de lactide [-CH(CH₃)-COO-] et des (ii) unités de glycolide [-CH₂-COO-] dans le(s) résidu(s) de poly(lactide-co-glycolide) est compris entre 1:99 et 12:88,
et dans lequel le poids moléculaire moyen en nombre du copolymère est d'au moins 20000 g/mol.

9. Le matériau selon l'une quelconque des revendications 1-8, qui est lyophilisé.

10. Le matériau selon l'une quelconque des revendications 1-9, dans lequel les pores du matériau sont occupés au moins partiellement par un ou plusieurs composants de la matrice extracellulaire.

11. Le matériau selon la revendication 10, dans lequel le composant de la matrice extracellulaire est sélectionné parmi le sulfate de chondroïtine, le hyaluronane, l'acide hyaluronique, le sulfate d'héparine, le sulfate d'héparane, le sulfate de dermatane, les facteurs de croissance, la thrombine, la fibrine, la fibronectine, l'élastine, le collagène, la gélatine et l'aggrécane.

12. Polymère biodégradable présentant la formule générale :
A-O-(CH₂CH₂O)ₙ-méthyl
dans lequel
A représente un résidu de poly(lactide-co-glycolide) ayant un poids moléculaire d'au moins 8000 g/mol, le rapport molaire des (i) unités de lactide [-CH(CH₃)-COO-] et des (ii) unités de glycolide [-CH₂-COO-] dans le résidu de poly(lactide-co-glycolide) étant compris entre 80:20 et 10:90,
n représente le nombre moyen d'unités -(CH₂CH₂O)- dans une chaîne polymère et est un nombre entier compris entre 16-250,
le rapport molaire des (iii) unités de polyéthylène-glycol [-(CH₂CH₂O)-] par rapport à la quantité combinée des (i) unités de lactide [-CH(CH₃)-COO-] et des (ii) unités de glycolide [-CH₂-COO-] dans le(s) résidu(s) de poly(lactide-co-glycolide) est compris entre 1:99 et 12:88,
et dans lequel le poids moléculaire moyen en nombre du copolymère est d'au moins 20000 g/mol.

13. Méthode de préparation d'un matériau poreux biodégradable composé d'un polymère, la méthode comprenant les étapes suivantes :
(a) dissolution d'un polymère tel que défini dans l'une quelconque des revendications 1-8 dans un solvant non aqueux afin d'obtenir une solution polymère ;
(b) congélation de la solution obtenue à l'étape (a) afin d'obtenir une solution polymère congelée ; et
(c) lyophilisation de la solution polymère congelée obtenue à l'étape (b) afin d'obtenir le matériau poreux biodégradable.

14. La méthode selon la revendication 13, dans laquelle la solution polymère, après l'étape (a), est versée ou coulée dans un moule approprié.

15. Elément de dispositif médical composé d'un matériau comprenant un polymère tel que défini dans la revendication 12.

16. L'élément de dispositif médical selon la revendication 15, dans lequel l'élément est le revêtement extérieur d'un dispositif médical.

17. Dispositif médical composé d'un matériau comprenant un polymère tel que défini dans la revendication 12.

18. Le dispositif médical selon la revendication 17, qui se présente sous la forme d'une grille ou d'un filet.

19. Le dispositif médical selon la revendication 17, qui se présente sous la forme d'une fibre.

20. Matériau poreux biodégradable tel que défini dans l'une quelconque des revendications 1-11 destiné à être utilisé dans les domaines de la thérapie, de la dentisterie ou de la chirurgie.

21. L'utilisation d'un matériau poreux biodégradable tel que défini dans l'une quelconque des revendications 1-11 pour la préparation d'un support permettant de supporter l'adhésion cellulaire ou la croissance interne pour la régénération des tissus.

22. L'utilisation d'un matériau poreux biodégradable tel que défini dans l'une quelconque des revendications 1-11 pour la préparation d'un pansement pour les plaies.
